# EUROPÄISCHE PATENTSCHRIFT

(11) **EP 0 625 908 B1**
(45) Veröffentlichungstag und Bekanntmachung des Hinweises auf die Patenterteilung: **17.12.1997**
(21) Anmeldenummer: 93903233.0
(22) Anmeldetag: 25.01.1993
(51) Int. Cl.: A61K 38/46, A61K 38/48, C12N 9/74

(54) **ANTIDOT FÜR HIRUDIN UND SYNTHETISCHE THROMBININHIBITOREN**
ANTIDOTE FOR HIRUDIN AND SYNTHETIC THROMBIN INHIBITORS
ANTIDOTE POUR L'HIRUDINE ET LES INHIBITEURS SYNTHETIQUES DE LA THROMBINE

(30) Priorität: 11.02.1992 DE 4203965
(43) Veröffentlichungstag der Anmeldung: 30.11.1994
(73) Patentinhaber: Max-Planck-Gesellschaft zur Förderung der Wissenschaften e.V., D-37073 Göttingen (DE)
(72) Erfinder: NOWAK, Götz, D-5083 Erfurt (DE); BUCHA, Elke, D-5032 Erfurt (DE)
(74) Vertreter: Weisert, Annekäte, Dipl.-Ing. Dr.-Ing.
(86) Internationale Anmeldenummer: EP9300162
(87) Internationale Veröffentlichungsnummer: WO9315757

(56) Entgegenhaltungen:
- BIOCHEMISTRY. Bd. 26, Nr. 3, 10. Februar 1987, EASTON, PA US Seiten 772 - 780 H. HOFMANN ET AL. 'BLOOD COAGULATION INDUCED BY THE VENOM OF BOTHROPS ATROX. I. IDENTIFICATION, PURIFICATION, AND PROPERTIES OF A PROTHROMBIN ACTIVATOR.'
- FASEB JOURNAL Bd. 3, Nr. 3, 9. Februar 1989, BETHESDA, MD US Seite A328 J. FAREED ET AL. 'DO WE NEED TO NEUTRALIZE HIRUDIN'S ANTICOAGULAT EFFECTS TO MINIMIZE BLEEDING?' in der Anmeldung erw hnt
- NAUNYN-SCHMIEDEBERG'S ARCHIVES OF PHARMACOLOGY. ABSTRACTS OF THE 33RD SPRING MEETING, MAINZ, 10-12 MARCH 1992 Bd. 345, Nr. SP.1, 1992, BERLIN, DE Seite R 98 G. NOWAK ET AL. 'HIRUDIN CONSUMPTION RESULTING FROM SNAKE VENOM-INDUCED ACTIVATION OF PROTHROMBIN IN RATS.'

## Beschreibung

Die Erfindung betrifft ein Antidot für Hirudin und synthethische Thrombininhibitoren, die Verwendung einer Verbindung, die Prothrombin zu Meizothrombin spaltet, eines Prothrombin-Intermediats oder eines pharmakologisch annehmbaren Salzes davon als Antidot für Hirudin und synthetische Thrombininhibitoren oder zur Herstellung eines Antidots für Hirudin und synthetische Thrombininhibitoren.

Antikoagulantien, wie beispielsweise Heparin oder Cumarine, finden heute zur Therapie und Prophylaxe thromboembolischer Erkrankungen, insbesondere zur Behandlung des Herzinfarkts, der Arteriosklerose, ferner bei Bluttransfusionen und nach Operationen vielfach Verwendung. Sie können auch als Mittel gegen Erfrierungen verwendet werden, da sie das bei großer Kälte eintretende Gerinnen des Blutes verhindern, durch welches sonst die Blutgefäße teilweise verstopft und die erfrorenen Organe vom Blutkreislauf abgeschnitten werden.

Hirudin, das aus der Speicheldrüse von Hirudo medicinalis (Blutegel) gewonnen wird, ist ein Antikoagulans, dessen Wirkung auf der Bildung einer chemischen Verbindung mit Thrombin beruht, wodurch dessen katalytische Wirkung inhibiert wird. Hirudin ist ein Miniprotein aus 65 Aminosäuren mit einem Molekulargewicht von 7 kD. Aufgrund seiner starken Affinität zu Thrombin (kᵢ-Werte von 10⁻¹² Mol/l) und seines direkten Wirkungsmechanismus ist es von großem Interesse. Seine klinische Anwendung war in der Vergangenheit äußerst beschränkt, da Hirudin nicht leicht in standardisierter Form zugänglich war und kein Gegenmittel verfügbar war. Hirudin kann heute gentechnologisch hergestellt werden und daher wäre seine klinische Anwendung in der nächsten Zeit möglich.

Beispielsweise werden in der EP-A-0 468 327 pharmazeutische Präparate für die orale Verabreichung beschrieben, die rekombinantes Hirudin enthalten.

Hirudin wurde in den letzten Jahren pharmakologisch intensiv untersucht und die pharmakologischen Daten wurden bei Versuchstieren und am Menschen erfaßt. Hirudin wird in der Leber nicht metabolisiert, sondern in unveränderter Form über die Nieren ausgeschieden. Hirudin wird mit einer Halbwertszeit von etwa 1 bis 2 Stunden aus dem Körper ausgeschieden und verteilt sich in den extrazellulären Flüssigkeitsräumen des Körpers. Ähnlich wie Heparin wird Hirudin nicht oral resorbiert. In früheren Untersuchungen wurde gezeigt, daß Hirudin in nahezu allen Thrombosemodellen wirksam ist, so auch beim Endotoxinschock und bei experimenteller Koronarthrombose sowie bei der Verhinderung der Reocclusion nach Thrombolyse. Bei klinisch-pharmakologischen Untersuchungen wurden keine immunologischen Reaktionen nachgewiesen. Hirudin hat sich bei klinischen Untersuchungen als Antikoagulans und Antithrombotikum als dem Heparin überlegen erwiesen.

In letzter Zeit haben synthetische Thrombininhibitoren große Bedeutung erlangt. Augenblicklich wird weltweit in vielen Forschungslaboratorien zu derartigen synthetischen Inhibitoren Synthesearbeit geleistet. Am weitesten fortgeschritten sind die Untersuchungen mit Derivaten des Benzamidins, wie z.B. dem kleinmolekularen synthetischen Thrombininhibitor NAPAP (Nα-(2-Naphthylsulforyl-glycyl)-D,L-amidinophenylalanin-piperidid) und mit sogenannten Tripeptiden. Alle synthetischen Thrombininhibitoren befinden sich augenblicklich in der präklinischen Untersuchung. Ihre Wirkungen sind qualitativ denen von Hirudin gleichzusetzen. Der Metabolismus der synthetischen Thrombininhibitoren unterscheidet sich jedoch von dem des Hirudins. In der Regel werden die Thrombininhibitoren in der Leber oder im Blut metabolisiert. Es ist abzusehen, daß in Kürze derartige Substanzen für die klinische Erprobung zur Verfügung stehen. Der Vorteil gegenüber dem Hirudin liegt vor allem darin, daß diese Substanzen oral verabreicht werden können.

Trotz der günstigen Wirkungen ist der klinische Einsatz von Hirudin bis jetzt unterblieben bzw. erfolgt nur sehr eingeschränkt, da, wie oben erwähnt, kein Antidot zur Verfügung steht, welches z.B. beim Heparin mit dem Protaminsulfat gegeben ist. Das Vorhandensein eines Antidots ist bei einem Thrombininhibitor zwingend erforderlich, denn bei zufälligen Überdosierungen oder bei Patienten mit Nierenfunktionsstörungen, bei denen eine Blutungskomplikation droht, muß sofort ein Antidot vorhanden sein, wenn eine Überdosierung festgestellt wird. Derartige Blutungskomplikationen treten beispielsweise als hämorrhagische Nebenwirkungen auf, vor allem in den Gefäßgebieten des Peritoneums, der Pleura, des Pericards und der Pia mater, aber auch in Operationsschnittwunden, wie dies an Tieren mit extrem hohen Blutspiegeln eines Thrombininhibitors beobachtet wurde.

In der Vergangenheit wurden verschiedene Antidotprinzipien für Hirudin experimentell untersucht, so der Einsatz von Gammathrombinpräparaten, wie DFP-Thrombin oder Benzoylthrombin (Brüggener, E., Walsmann, P., Markwardt, F.: Neutralization of hirudin anticoagulant action by DIP-thrombin. Pharmazie 1989; 44: 648-9). Diese Präparate haben sich jedoch in der Praxis nicht bewährt, da ihre Affinität zu Hirudin zu gering ist und sie zu toxisch sind. Auch aktivierte Plasmafraktionen, wie FEIBA oder Autoplex-Präparate, sind wegen der thromboplastinähnlichen Aktivität ungeeignet (Fareed. J., Walenga, J.M.: Do we need to neutralize hirudin's anticoagulant effects to minimized bleeding. Fed Proc 1989; 3: A328; Walenga, J.M., Piffarre, R., Hoppensteadt, D.A., Fareed: Development of recombinant hirudin as a therapeutic anticoagulant and antithrombotic agent. Some objective considerations. Sem Thromb Hemost 1989; 15: 316-33).

Der vorliegenden Erfindung liegt die Aufgabe zugrunde, ein Antidot für Hirudin und synthetische Thrombininhibitoren zur Verfügung zu stellen. Das Antidot soll gut wirksam sein, d.h. eine ausreichende Affinität zu Hirudin und synthetischen Thrombininhibitoren aufweisen, seine Dosierung soll einfach sein und es soll leicht zugänglich sein.

Gegenstand der Erfindung ist ein Antidot für Hirudin und synthetische Thrombininhibitoren, das dadurch gekennzeichnet ist, daß es eine Verbindung, die Prothrombin zu Meizothrombin spaltet, ein Prothrombin-Intermediat, ein pharmakologisch annehmbares Salz davon oder ein Gemisch dieser Verbindungen zusammen mit üblichen Trägerstoffen und/oder Verdünnungsmitteln enthält, wobei es als Prothrombin-Intermediat Meizothrombin, PIVKA-Prothrombin, Meizothrombin-des-Fragment-1 oder eine Meizothrombin enthaltende Verbindung enthält.

Gegenstand der Erfindung ist weiterhin die Verwendung einer Verbindung, die Prothrombin zu Meizothrombin spaltet, eines vorstehend definierten Prothrombin-Intermediats, eines pharmakologisch annehmbaren Salzes davon oder eines Gemisches dieser Verbindungen zusammen mit üblichen Trägerstoffen und/oder Verdünnungsmitteln als Antidot für Hirudin und synthetische Thrombininhibitoren oder zur Herstellung eines Antidots für Hirudin und synthetische Thrombininhibitoren.

Als Thrombininhibitoren kommen erfindungsgemäß Hirudin und synthetische, vorzugsweise kleinmolekulare, Thrombininhibitoren in Frage. Beispiele für synthetische Thrombininhibitoren sind NAPAP (Nα-(2-Naphtylsulforyl-glycyl)-D,L-amidinophenylalanin-piperidid), weiterhin die Derivate des Tripeptids Phe-Pro-Arg, wie Boronsäure-Derivate, Argininale, Chlormethylketon-Derivate und Aminosäure-modifizierte Derivate sowie Benzamidin-Derivate und auch sogenannte Hirologe, d.h. synthetische Hirudin-analoge Teilsequenzen. Bei all diesen Verbindungen ist das Antidotprinzip das gleiche wie bei Hirudin.

Das erfindungsgemäße Antidot liegt in einer für die parenterale Verabreichung geeigneten Form, d.h. für die subkutane, intramuskuläre oder intravenöse Verabreichung geeigneten Form, vor. Die intravenöse Verabreichung ist bevorzugt. Gegebenenfalls kann das erfindungsgemäße Antidot auch als Dauerinfusion verabreicht werden.

Erfindungsgemäß wird als Verbindung, die Prothrombin zu Meizothrombin spaltet, ein Schlangengift verwendet. Beispiele für Schlangengifte sind Ecarin und die Gifte von Dispholidus-, Rhabdophis-, Bothrops-, Notechis-, Oxyuranus- und Russell-Vipern-Arten. Bevorzugt ist Ecarin, eine gereinigte Giftfraktion aus Echis-carinatus-Toxin. Die Schlangengifte sind als Biochemikalien sämtlich, beispielsweise bei der Firma SIGMA Chemie GmbH, 8024 Deisenhofen, zu erwerben. Gereinigte Fraktionen einzelner aufgeführter Schlangengifte können auf Anfrage bei der Firma Pentapharm in der Schweiz bezogen werden. Die Schlangengifte, insbesondere Ecarin sowie immobilisiertes Ecarin, können ebenfalls von der Firma Pentapharm in der Schweiz käuflich bezogen werden.

Die Gifte sind als Trockensubstanz, in der Regel gefriergetrocknet, in Mengen zwischen 5 mg und 1 g erhältlich. Alle Gifte sind gut wasserlöslich und sollten mit 0,9%iger Kochsalzlösung aufgenommen werden. Die Menge der Dosis kann vom Fachmann leicht bestimmt werden. Die Dosis hängt von dem Körpergewicht, dem Hirudingehalt und dem Verabreichungsweg ab. Die verwendeten Mengen liegen für einen Menschen mit 70 kg Körpergewicht im Bereich von 0,5 bis 5 mg.

Erfindungsgemäß wird als Prothrombin-Intermediat Meizothrombin, Meizothrombin-des-Fragment-1, PIVKA-Prothrombin oder eine Meizothrombin enthaltende Verbindung verwendet. Meizothrombin ist im Handel erhältlich und kann ebenfalls von der oben genannten Firma Pentapharm bezogen werden. Meizothrombin, PIVKA-Prothrombin, Meizothrombin-des-Fragment-1 oder andere Prothrombin-Intermediate können aber auch in vitro gebildet werden.

Vier Faktoren des Gerinnungssystems, Faktor II (Prothrombin), Faktor VII, Faktor IX und Faktor X sind, wie im folgenden Schema dargestellt, dadurch gekennzeichnet, daß sie gamma-Carboxyglutaminsäurereste enthalten. Diese gamma-Carboxylierung an der Glutaminsäure erfolgt erst nach der ribosomalen Synthese des "Acarboxy-Faktors" in der Leber mit Hilfe eines Enzymsystems, welches Vitamin K als Cofaktor benötigt.

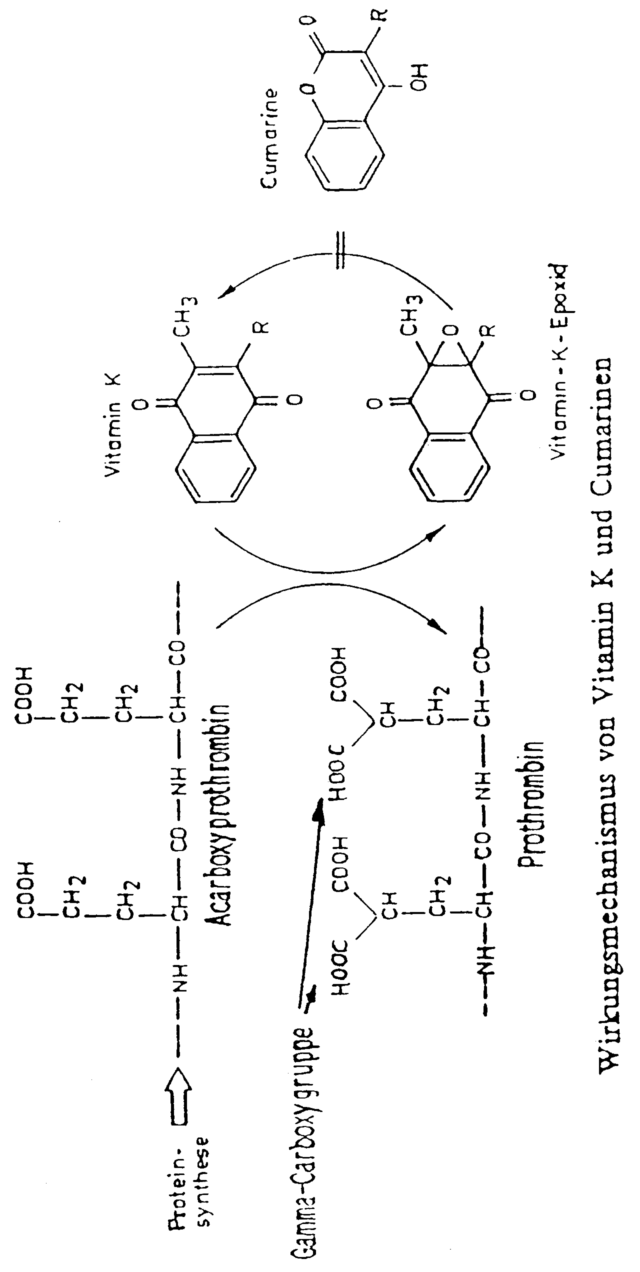

Die gamma-Carboxyglutaminsäurereste sind für die Gerinnungswirkung essentiell. Sie stellen die nötigen Bindungsvalenzen für Calciumionen dar. Bei Behandlung mit indirekten Antikoagulantien vom Typ der Dicumarole ("Vitamin-K-Antagonisten") kann die postribosomale gamma-Carboxylierung nicht stattfinden und es befinden sich im Blut inkomplette Gerinnungsfaktoren bzw. Acarboxy-Faktoren, denn ihnen fehlen die Calcium-bindenden gamma-Carboxy-Gruppen. Diese Gerinnungsfaktoren werden auch PIVKA-Faktoren (PIVKA = proteins induced by Vitamin K antagonists) genannt.

Wenn Plasma von Patienten, die mit derartigen Antikoagulantien vom Dicumarol-Typ behandelt wurden, mit Ecarin versetzt wird, dann entsteht in diesem Plasma aus dem PIVKA-Prothrombin PIVKA-Meizothrombin in der gleichen Weise durch eine limitierte Proteolyse wie dies auch in normalen Plasmaproben mit Prothrombin geschieht.

Dieses PIVKA-Meizothrombin bzw. andere PIVKA-Intermediate haben ihre Bindungsfähigkeit zu Hirudin behalten, sie haben aber keine bzw. wesentlich geringere Wirkungen auf andere Faktoren der Gerinnungskaskade (Plättchen, Fibrinogen, Thrombomodulin u.a.). Erfindungsgemäß können Meizothrombin, PIVKA-Meizothrombin, deren Intermediate und PIVKA-Intermediate aus PIVKA-Prothrombin als Antidot verwendet werden. Diese können vom Menschen oder von anderen Säugetieren stammen.

Zur Herstellung von Meizothrombin kann beispielsweise immobilisiertes Ecarin (Produkt der Pentapharm AG, Basel) in Minisäulen in einer Größe von beispielsweise 2 - 4 cm³ gepackt werden. Ecarin-Immobilisat gelangt in gequollenem Zustand, suspendiert in einer wäßrigen Lösung von Natriumchlorid 0,15 M, Natriumacetat 0,02 M, Prionex® (Warenzeichen der Pentapharm AG für eine proteinstabilisierende Polypeptidfraktion aus gereinigtem Schweinehaut-Kollagen) 0,2% und Trichlorisobutanol 0,3%, pH 5,5, zur Auslieferung. 1 g gequollenes Ecarin-Immobilisat produziert aus Bariumcitrat-Eluat bei 37°C, pH 8,4, innerhalb von 30 min 500 bis 700 U amidolytische Aktivität (1 U = 123 NIH-Einheiten), gemessen an Tos-Gly-Pro-Arg-pNA (Chromozym® TH).

Dann werden gereinigte Prothrombinfraktionen auf diese Säulen gegeben, und das gebildete Meizothrombin, gegebenenfalls nach Stabilisierung mit Heparin, wird anschließend gefriergetrocknet. Das gefriergetrocknete Material wird steril in Ampullen abgepackt und dann für die Verwendung als Antidot mit einem geeigneten Lösungsmittel, vorzugsweise mit steriler Natriumchloridlösung, die für die intravenöse Injektion geeignet ist, rekonstituiert.

Zur Herstellung von Meizothrombin-des-Fragment-1 wird dasselbe Verfahren wie für Meizothrombin verwendet. Im Batch-Verfahren ist nur eine längere Reaktionszeit (3 - 4 h) vorzusehen. Meizothrombin-des-Fragment-1 ist ein Folgeprodukt der Meizothrombin-Aktivierung.

Für die parenterale Verabreichung kann das Antidot für die Injektion, wie die intravasale, z.B. intravenöse, intramuskuläre oder subkutane, Injektion, formuliert werden. Die intravasale Verabreichung ist bevorzugt. Zubereitungen für die Injektion können in Eindosenform, zum Beispiel in Ampullen, oder in Mehrfachdosis-Behältern mit einem zugegebenen Konservierungsstoff vorliegen. Die Zubereitungen können in Form von Suspensionen, Lösungen oder Emulsionen in öligen oder wäßrigen Trägern vorliegen und Zubereitungshilfsmittel enthalten, wie Suspendier-, Stabilisier- und/oder Dispersionsmittel, und/oder Mittel zur Einstellung der Tonizität der Lösung. Alternativ kann der aktive Bestandteil in Pulverform für die Konstitution mit einem geeigneten Träger, zum Beispiel sterilem pyrogenfreien Wasser, vor der Verwendung vorliegen.

In Ampullen und Mehrfachdosis-Behältern zur intravasalen Anwendung sollte das Produkt, z B. das Meizothrombin, vorzugsweise gefriergetrocknet vorliegen. In dieser Form ist es in sterilem Wasser, physiologischer Kochsalzlösung oder Pufferlösungen, die Ca⁺⁺-Ionen enthalten und auf einen pH-Wert von 6,5 - 7 eingestellt sind, vollständig lösbar. Die Ampullen sollten 0,5; 1; 2 oder 5 mg Meizothrombin enthalten, Mehrfachdosis-Behälter 10; 20 oder 50 mg. Eine kurzfristige Lagerung (Tage - 1 Monat) ist auch in tiefgefrorenem Zustand (-25°C) möglich. Zur extravasalen parenteralen Applikation kommen auch Mischungen mit 1% Dimeticon-Suspensionen, 5% Erythrozytenmembran-Fragmenten oder Bariumsulfat-Emulsionen in Betracht. Auch eine Liposomenadsorption ist zur extravasalen Anwendung geeignet. Der Gehalt der Einzelformulierung an Meizothrombin ist bei diesen Präparationen den Ampullen- und Mehrfachdosis-Abfüllungen wie für die intravasale Anwendung gleichzusetzen.

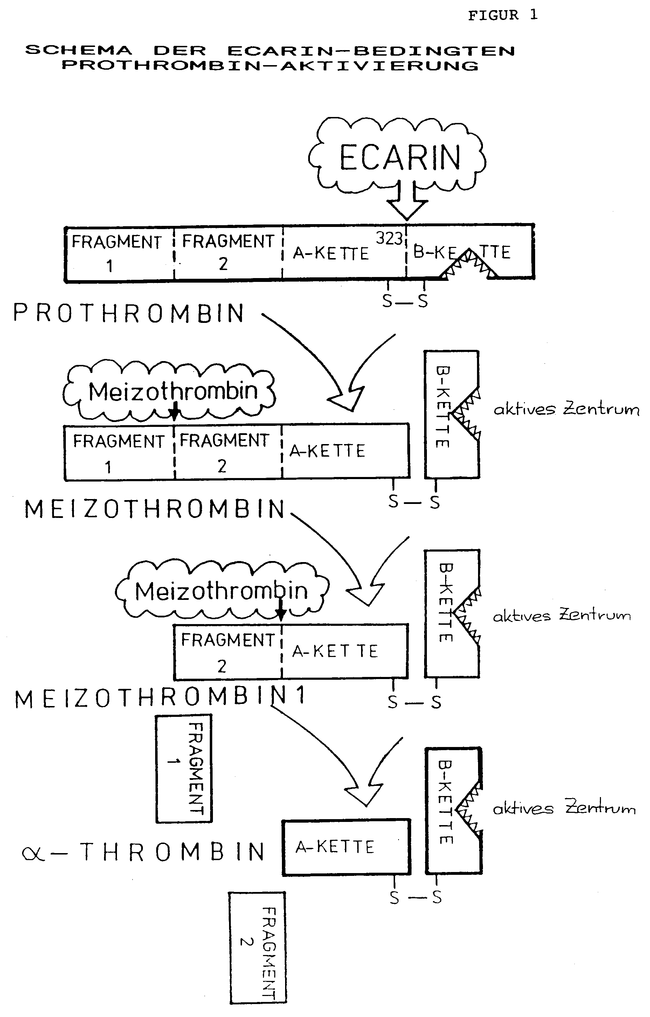

Erfindungsgemäß wird als Schlangengift bevorzugt Ecarin, eine hochgereinigte Fraktion des Echis-carinatus-Toxins, verwendet. Ecarin spaltet am Arginin 323 des Prothrombins eine Peptidbindung, wodurch das Intermediat Meizothrombin entsteht. Normalerweise erfolgt die weitere Umsetzung durch Autokatalyse bzw. durch Thrombinakzeleration. Bei Vorliegen von Hirudin oder synthetischen Thrombininhibitoren im Plasma kommt es zur Interaktion zwischen Meizothrombin und dem Inhibitor. Im Gegensatz dazu kann Heparin nicht mit Meizothrombin reagieren. Diese Wirkungen sind in der beigefügten Figur 1 dargestellt.

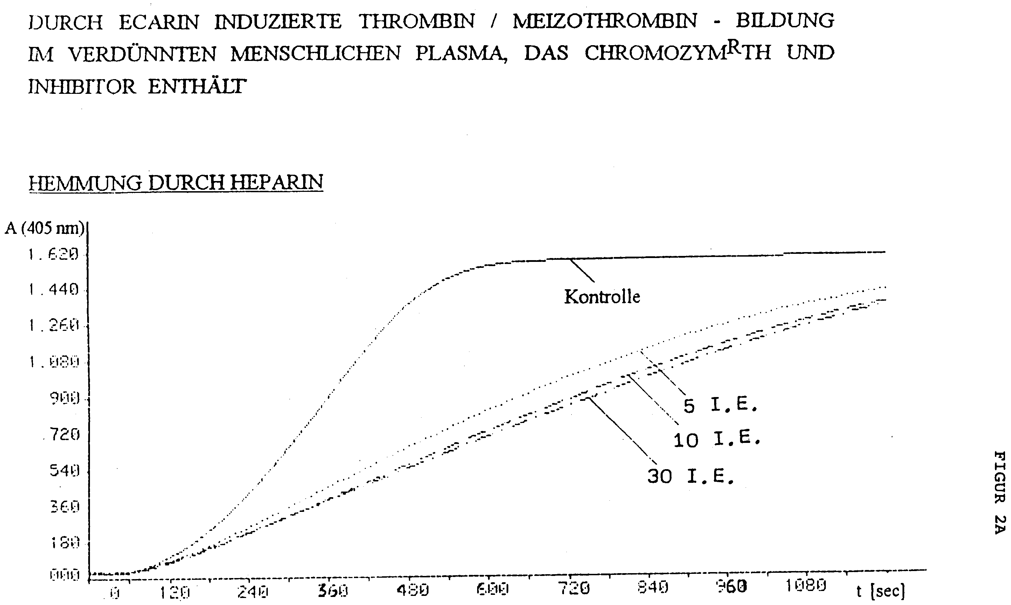

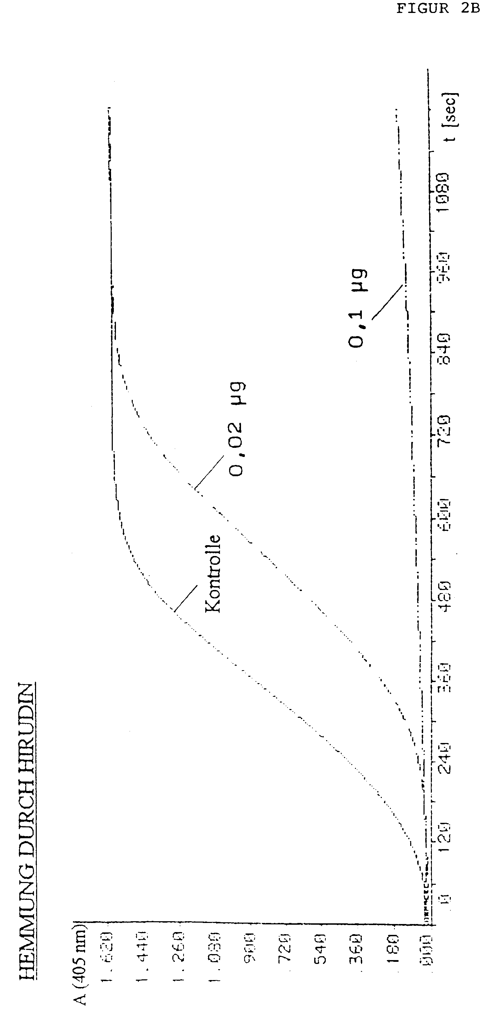

Es konnte gezeigt werden, daß im stark verdünnten Humanplasma durch Ecarin die Prothrombin-Aktivierung induziert wird. Die Thrombin-/Meizothrombin-Aktivität wurde mit Chromozym® TH gemessen. Die erhaltenen Ergebnisse sind in Figur 2 dargestellt. Die bei Heparinmengen von 2,5 - 35 IE/ml nachweisbare dosisunabhängige Restaktivität entspricht dem Ausmaß der Meizothrombinbildung nach Ecarineinwirkung. Hirudin hemmt dosisabhängig komplett die Meizothrombin-/Thrombinbildung.

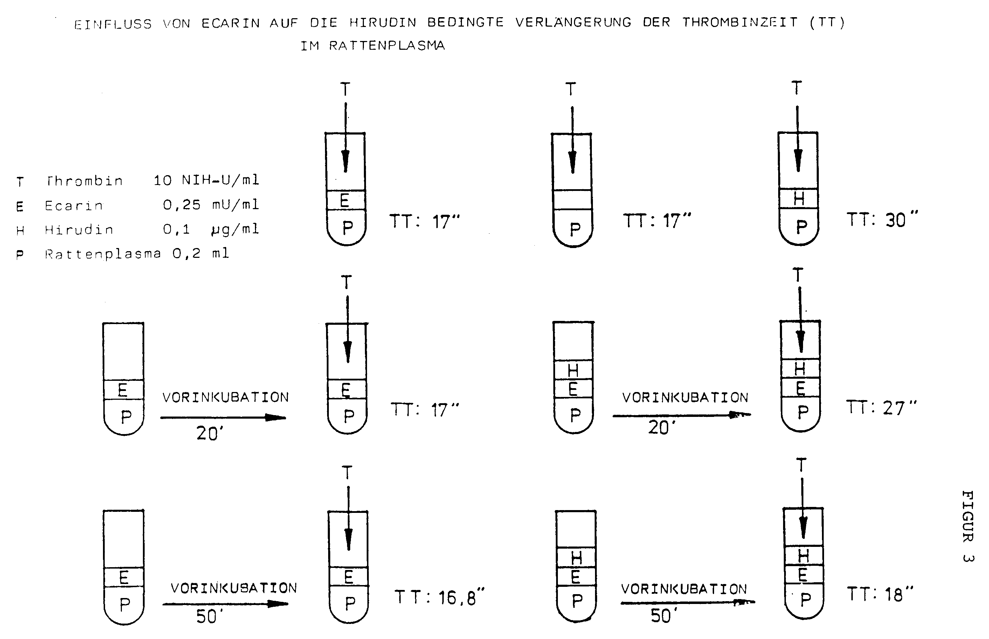

Zum Nachweis der Antidotwirkung wurden verschiedene pharmakologische Versuche durchgeführt. Die Antidotwirkung des erfindungsgemäßen Antidots wurde im Rattenversuch nachgewiesen. In der beigefügten Figur 3 sind die erhaltenen Ergebnisse dargestellt. Rattenzitratplasma wurde mit Thrombin zur Gerinnung gebracht. Die Kontrollen haben Gerinnungszeiten von durchschnittlich 17 sec. Werden dem Versuchsansatz 0,1 µg Hirudin/ml zugesetzt, ist die Thrombinzeit auf 30 sec verlängert. Wenn der Ansatz mit 0,25 mU Ecarin/ml vorinkubiert wird, kommt es nach einer Vorinkubationsdauer von 20 sec zu einer Verkürzung der Thrombinzeit auf 27,5 sec durch den Verbrauch von Hirudin im Plasma. Bei einer 50 sekündigen Vorinkubation entspricht die Thrombinzeit dem Kontrollwert (ohne Hirudinzusatz). Die verwendete Ecarinkonzentration selbst bewirkt in diesem Zeitbereich keine Gerinnungsbeschleunigung im Versuchsansatz.

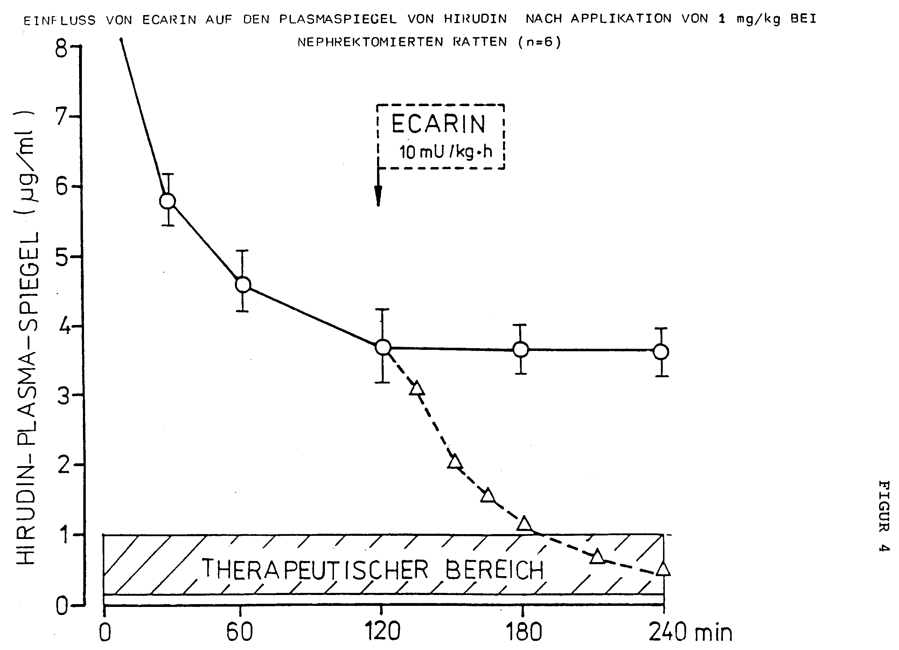

Zum Nachweis der Antidotwirkung wurden weiterhin in nephrektomierten Ratten konstante Blutspiegel von Hiurdin im Bereich zwischen 3,5 - 4,2 µg/ml nach intravenöser Applikation von 1 mg/kg Hirudin erzeugt. Während der Infusion von 50 µg Ecarin/kg·h⁻¹ fällt der Hirudinspiegel rasch ab und ist bereits 30 min nach Applikationsbeginn signifikant auf 2,1 µg/ml vermindert. Am Ende der Toxininfusion ist der Blutspiegel auf 1,2 µg/ml abgefallen. Rebound-Phänomene sind nicht zu beobachten. Als besonders bedeutsam ist der Befund zu werten, daß bei diesen Versuchen die Plättchenzahl sowie der Fibrinogenspiegel nahezu unverändert blieben. Auch bei Verkürzung der Ecarin-Infusionsdauer auf 30 bzw. 15 min, und damit einer Dosisreduktion um die Hälfte bzw. auf ein Viertel, ist dieser Hirudinspiegelabfall in ähnlicher Weise nachzuweisen (vgl. Figur 4).

In einer weiteren Versuchsreihe wurde der Antidotmechanismus an einem Blutungsmodell nachgewiesen. Hierzu wurden nephrektomierten Ratten 5 mg/kg Hirudin intravenös appliziert. Nach 2 Stunden war ein konstanter Blutspiegel von 18 µg/ml Hirudin erreicht. Zu diesem Zeitpunkt wurde eine Blutungszeit von länger als 100 min gemessen. Wird den Ratten Echis-carinatus-Toxin (1 mg/kg·h⁻¹) infundiert, dann sistiert die Blutung nach 90 min. Der Blutverlust aus der experimentellen Schnittwunde ist stark vermindert, der Hirudin-Blutspiegel ist auf Werte von 1 - 3 µg/ml gefallen. Die erhaltenen Ergebnisse sind in der folgenden Tabelle aufgeführt.

| Ecarin-Antagonismus gegen Hirudin-induzierte Blutung bei Ratten | | | | |
|---|---|---|---|---|
| Zeit (h) | Versuchsprotokoll | Plasmaspiegel an Hirudin (µg/ml) | Blutungszeit* (min) | Blutverlust |
| 0 | bilaterale Nephrektomie | | | |
| 2 | | 0 | 2,52 | - |
| 2 | Hirudin i.v. (5 mg/kg) | | | |
| 4 | | 17,8 | > 30 | +++ |
| 4 | E.carinatus-Toxin-Infusion, (1 mg/kg·h⁻¹), Prothrombin-Substitution | | | |
| 5 | | 5,9 | 8,17 | (+) |
| 6 | | 3,5 | 6,40 | - |

| | | | | |
|---|---|---|---|---|
| (* Einschnitt in die Bauchhaut) | | | | |

Aus den obigen Versuchen geht eindeutig hervor, daß Meizothrombin, ein intermediäres Prothrombin-Aktivierungsprodukt von Ecarin, als Antidot bei Hirudinintoxikationen wirkt. Ähnliche pharmakologische Ergebnisse wurden mit anderen Prothrombin-Intermediaten erhalten.

Die folgenden Beispiele erläutern die Erfindung.

### BEISPIEL 1

### Präparation von Meizothrombin im Batchverfahren

500 ml Prothrombin-Lösung:
Aus 2 l Oxalatplasma wird Prothrombin mittels Ba SO₄ gefällt und anschließend mit 0,1 M Natriumoxalat und 0,006 M Natriumcitrat bei pH 7,5 gewaschen. Nach Eluierung des Prothrombins mit 0,15 M Natriumcitrat und pH-Regulierung auf 7,5 wird bei -5°C eine Alkoholfällung (19%) durchgeführt. Der Überstand wird auf pH 5,5 eingestellt und die Alkoholkonzentration auf 25% erhöht. Der Niederschlag wird gelöst und für 5 min auf 50°C erhitzt, bei 6000 g zentrifugiert und dann mit Acetat-Puffer auf 500 ml aufgefüllt.

50 mg humanes Prothrombin gelöst in 500 ml Acetat-Puffer pH 5,5 werden mit 10 g immobilisiertem Ecarin 60 min bei 20°C gerührt. Nach Zentrifugation (15 min bei 6000 g) wird der Überstand mit Hilfe einer Eichkurve, die mit einem Meizothrombin-Standard erhalten wurde, kalibriert auf 1 mg/ml, in 10-ml-Ampullen gefüllt, gefriergetrocknet und verschweißt. Die Abfüllung erfolgt bei gleichzeitiger Sterilisation unter aseptischen Bedingungen. Nach Gefriertrocknung erfolgt der Verschluß der Ampullen und die Lagerung bei 4°C. In dieser Form sind die Präparate mindestens 12 Monate ohne Aktivitätsverlust lagerbar.

### BEISPIEL 2

### Präparation von PIVKA-Meizothrombin im Säulenverfahren

250 ml Essigsäure-Puffer-Lösung (pH 5,5), die 25 mg PIVKA-Prothrombin (Präparation nach Beispiel 1, Ausgangsmaterial 1 l Oxalatplasma, gewonnen durch Pooling vom Plasma Dicumarol-behandelter Patienten) enthält, wird über 40 cm³-Säulen (50 - 75 cm Länge), gepackt mit 10 g gequollenem Ecarin-Immobilisat, eluiert. Das eluierte Volumen wird auf 500 ml begrenzt, danach sterilfiltriert (mit Sterilfiltern) und danach in 10 ml-Portionen ampulliert. Nach der Gefriertrocknung werden die Ampullert verschlossen und in gleicher Weise wie in Beispiel 1 beschrieben gelagert.

### BEISPIEL 3

Die lyophilisierten 1-mg-Ampullen werden mit 10 ml 0,9% NaCl gelöst und in dieser Form appliziert. Zur intravenösen Infusion werden Mehrfachdosis-Behälter, 10 mg Meizothrombin enthaltend, mit 0,9% NaCl gelöst und in jeweils 500 ml 0,9% NaCl-Infusionslösung appliziert. Die Infusionsgeschwindigkeit sollte etwa 1000 ml/h betragen. Der Plasma-Hirudingehalt muß kontinuierlich mit einer bed-side-Methode kontrolliert werden.

## Patentansprüche

1. Antidot für Hirudin und synthetische Thrombininhibitoren, dadurch **gekennzeichnet,** daß es eine Verbindung, die Prothrombin zu Meizothrombin spaltet, ein Prothrombin-Intermediat, ein pharmakologisch annehmbares Salz davon oder ein Gemisch dieser Verbindungen zusammen mit üblichen Trägerstoffen und/oder Verdünnungsmitteln enthält, wobei es als Prothrombin-Intermediat Meizothrombin, PIVKA-Prothrombin, Meizothrombin-des-Fragment-1 oder eine Meizothrombin enthaltende Verbindung enthält.

2. Antidot nach Anspruch 1, dadurch **gekennzeichnet,** daß es als Verbindung, die Prothrombin zu Meizothrombin spaltet, ein Schlangengift enthält.

3. Antidot nach Anspruch 2, dadurch **gekennzeichnet,** daß es als Schlangengift Ecarin enthält.

4. Antidot nach mindestens einem der Ansprüche 1 bis 3 , dadurch **gekennzeichnet,** daß es in einer für die parenterale Verabreichung geeigneten Form vorliegt.

5. Antidot nach Anspruch 4, dadurch **gekennzeichnet,** daß es in einer für die subkutane, intramuskuläre oder intravasale Verabreichung geeigneten Form vorliegt.

6. Verwendung einer Verbindung, die Prothrombin zu Meizothrombin spaltet, eines Prothrombin-Intermediats, eines pharmakologisch annehmbaren Salzes davon oder eines Gemisches dieser Verbindungen zusammen mit üblichen Trägerstoffen und/oder Verdünnungsmitteln zur Herstellung eines Antidots für Hirudin und synthetische Thrombininhibitoren, wobei als Prothrombin-Intermediat Meizothrombin, PIVKA-Prothrombin, Meizothrombin-des-Fragment-1 oder eine Meizothrombin enthaltende Verbindung verwendet wird.

7. Verwendung nach Anspruch 6 dadurch **gekennzeichnet,** daß als Verbindung, die Prothrombin zu Meizothrombin spaltet, ein Schlangengift verwendet wird.

8. Verwendung nach Anspruch 7, dadurch **gekennzeichnet,** daß als Schlangengift Ecarin verwendet wird.

9. Verwendung nach mindestens einem der Ansprüche 6 bis 8, dadurch **gekennzeichnet,** daß ein für die parenterale Verabreichung geeignetes Antidot hergestellt wird.

## Claims

1. Antidote to hirudin and to synthetic thrombin inhibitors, characterised in that it contains a compound which splits prothrombin to meizothrombin, a prothrombin intermediate, a pharmacologically acceptable salt thereof or a mixture of these compounds together with conventional carriers and/or diluents, the prothrombin intermediate contained being meizothrombin, PIVKA-prothrombin, meizothrombin of fragment 1, or a compound containing meizothrombin.

2. Antidote according to claim 1, characterised in that it contains a snake venom as the compound which splits prothrombin to meizothrombin.

3. Antidote according to claim 2, characterised in that it contains ecarin as the snake venom.

4. Antidote according to at least one of claims 1 to 3, characterised in that it is in a form suitable for parenteral administration.

5. Antidote according to claim 4, characterised in that it is in a form suitable for subcutaneous, intramuscular or intravasal administration.

6. Use of a compound which splits prothrombin to meizothrombin, of a prothrombin intermediate, of a pharmaceutically acceptable salt thereof or of a mixture of these compounds together with conventional carriers and/or diluents for the preparation of an antidote to hirudin and to synthetic thrombin inhibitors, with meizothrombin, PIVKA-prothrombin, meizothrombin of fragment 1, or a compound containing meizothrombin being used as the prothrombin intermediate.

7. Use according to claim 6, characterised in that a snake venom is used as the compound which splits prothrombin to meizothrombin.

8. Use according to claim 7, characterised in that ecarin is used as the snake venom.

9. Use according to at least one of claims 6 to 8, characterised in that an antidote suitable for parenteral administration is prepared.

## Revendications

1. Antidote contre l'hirudine et les inhibiteurs synthétiques de la thrombine, caractérisé en ce qu'il contient un composé qui décompose la prothrombine en meizothrombine, un composé intermédiaire de la prothrombine, un sel pharmacologiquement acceptable de celui-ci ou un mélange de ces composés conjointement avec des véhicules et/ou des diluants usuels, le composé intermédiaire de prothrombine qui est contenu étant la meizothrombine, la PIVKA-prothrombine, la meizothrombine-des-fragment 1 ou un composé contenant de la meizothrombine.

2. Antidote selon la revendication 1, caractérisé en ce qu'il contient un venin de serpent comme composé qui décompose la prothrombine en meizothrombine.

3. Antidote selon la revendication 2, caractérisé en ce qu'il contient de l'écarine comme venin de serpent.

4. Antidote selon au moins l'une des revendications 1 à 3, caractérisé en ce qu'il se présente sous une forme appropriée à une administration parentérale.

5. Antidote selon la revendication 4, caractérisé en ce qu'il se présente sous une forme appropriée à une administration par voie sous-cutanée, intramusculaire ou intravasale.

6. Utilisation d'un composé qui décompose la prothrombine en meizothrombine, d'un composé intermédiaire de la prothrombine, d'un sel pharmacologiquement acceptable de celui-ci ou d'un mélange de ces composés conjointement avec des véhicules et/ou des diluants usuels, pour la préparation d'un antidote contre l'hirudine et les inhibiteurs synthétiques de la thrombine, le composé intermédiaire de la prothrombine qu'on utilise étant la meizothrombine, la PIVKA-prothrombine, la meizothrombine-des-fragment 1 ou un composé contenant de la meizothrombine.

7. Utilisation selon la revendication 6, caractérisée en ce qu'on utilise un venin de serpent comme composé qui décompose la prothrombine en meizothrombine.

8. Utilisation selon la revendication 7, caractérisée en ce qu'on utilise l'écarine comme venin de serpent.

9. Utilisation selon au moins l'une des revendications 6 à 8, caractérisée en ce qu'on prépare un antidote approprié à une administration parentérale.
